(19)

Europäisches
Patentamt
European
Patent Office
Office européen
des brevets

(11) **EP 3 086 250 A2**

(12) **EUROPEAN PATENT APPLICATION**

(43) Date of publication:
**26.10.2016 Bulletin 2016/43**

(51) Int Cl.:
***G06F 19/00*** *(2011.01)*    ***A61B 5/044*** *(2006.01)*

(21) Application number: **16165746.5**

(22) Date of filing: **18.04.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **20.04.2015   JP 2015085520**

(71) Applicant: **Nihon Kohden Corporation
Shinjuku-ku
Tokyo (JP)**

(72) Inventors:
• **Miyamura, Takahiro
  Tokyo (JP)**
• **Enomoto, Yoshinori
  Tokyo (JP)**

(74) Representative: **Hoffmann Eitle
Patent- und Rechtsanwälte PartmbB
Arabellastraße 30
81925 München (DE)**

(54) **PORTABLE MEDICAL APPARATUS, PROGRAM, AND METHOD OF DISPLAYING VITAL SIGNS INFORMATION**

(57)    A portable medical apparatus includes: a measuring section which is configured to measure a plurality of vital signs parameters based on vital signs signals of a patient; an operating section which is configured to receive a user operation; a displaying section which is configured to display: a first screen for displaying first information of at least a part of the plurality of vital signs parameters; and a second screen for displaying second information of at least a part of the plurality of vital signs parameters, the second information at least a part of which is different from the first information; and a controller which is configured to switch the first screen and the second screen in the displaying section, based on the user operation.

*FIG. 1*

EP 3 086 250 A2

**Description**

BACKGROUND

The presently disclosed subject matter relates to a portable medical apparatus, a program, and a method of displaying vital signs information.

Recently, a medical telemeter which can perform bidirectional wireless communication with a receiver is widely used. A medical telemeter is a small medical apparatus which is to be carried by a patient. A medical telemeter measures vital signs parameters (an electrocardiogram, the respiratory waveform, the heart rate, the respiratory rate, and the like) of a patient. Then, the medical telemeter displays the measured vital signs parameters on a display screen, and transmits the vital signs parameters to a receiver.

In a patient monitor such as a medical telemeter, it is preferable that the user (the doctor or the like) can recognize correctly and quickly information of necessary vital signs parameters. As a display technique for enabling information of vital signs parameters to be recognized correctly and quickly, JP-T-2005-529396 discloses a medical monitor. In the medical monitor disclosed in JP-T-2005-529396, the size of a region for displaying various kinds of patient information to be displayed (such as the blood pressure and the body temperature) and biological waveforms is made variable, and the size and the like of characters for displaying measurement values are changed in accordance with the size of the region (Fig. 3 and the like of JP-T-2005-529396).

In a medical telemeter, in order to respond to a request for miniaturizing the telemeter body, a small size screen is often employed. Therefore, such a medical telemeter displays a part of measured vital signs parameters on the display screen, and does not display the other vital signs parameters on the display screen unless the setting is changed. When the doctor or the like wishes to refer to detailed biological waveforms or the like, consequently, the doctor checks necessary information on a screen of a central monitor. Alternatively, the doctor changes the setting of the medical telemeter, and then refers to waveforms and the like which are displayed on the medical telemeter.

As described above, a medical telemeter measures vital signs parameters (vital signs) of a patient. Since information of vital signs parameters which can be displayed on a display screen is limited, the doctor or the like must know the detailed conditions of the patient through a central monitor, or manually perform complicated setting such as a change of display setting. However, it is often that the physical position of the patient wearing a medical telemeter is remote from that of a central monitor. Therefore, it is preferred that information of necessary vital signs parameters can be immediately referred from a screen of a medical telemeter without loss of visibility, and without the burden of changing the setting.

The above-described technique disclosed in JP-T-2005-529396 is mainly directed to a bedside monitor in which many vital signs parameters are displayed on one screen. In the case where the number of vital signs parameters to be displayed on one screen is increased, respective display regions are narrowed. When the technique disclosed in JP-T-2005-529396 is applied to an apparatus having a small screen such as a medical telemeter (portable medical apparatus), therefore, the visibilities of measurement values and waveforms of vital signs parameters are lowered.

SUMMARY

The presently disclosed subject matter may provide a portable medical apparatus, program, and method of displaying vital signs information in which information of various vital signs parameters can be checked without performing complicated setting.

The portable medical apparatus may comprise: a measuring section which is configured to measure a plurality of vital signs parameters based on vital signs signals of a patient; an operating section which is configured to receive a user operation; a displaying section which is configured to display: a first screen for displaying first information of at least a part of the plurality of vital signs parameters; and a second screen for displaying second information of at least a part of the plurality of vital signs parameters, the second information at least a part of which is different from the first information; and a controller which is configured to switch the first screen and the second screen in the displaying section, based on the user operation.

BRIEF DESCRIPTION OF THE DRAWINGS

Fig. 1 is a block diagram showing the configuration of a medical telemeter 10 (portable medical apparatus) of Embodiment 1.
Figs. 2A and 2B are conceptual views showing a display control on a displaying section 160 (touch panel) in Embodiment 1.

Fig. 3 is a flowchart showing the operation of a controller 150 in Embodiment 1.

Fig. 4 is a conceptual view showing a display control on a displaying section 160 (touch panel) in Application Example 2.

Fig. 5 is a flowchart showing the operation of a controller 150 in Application Example 2.

Fig. 6 is a conceptual view showing a display control on a displaying section 160 (touch panel) in Application Example 3.

Fig. 7 is a flowchart showing the operation of a controller 150 in Application Example 3.

Figs. 8A and 8B are conceptual views showing a display control on a displaying section 160 (touch panel) in Application Example 4.

DETAILED DESCRIPTION OF EXEMPLARY EMBODIMENTS

<Embodiment 1>

Hereinafter, the presently disclosed subject matter will be described by way of an embodiment thereof. However, the following embodiment is not intended to limit the presently disclosed subject matter as defined in the appended claims, and all combinations of features described in the embodiment are not always essential to solving means of the presently disclosed subject matter.

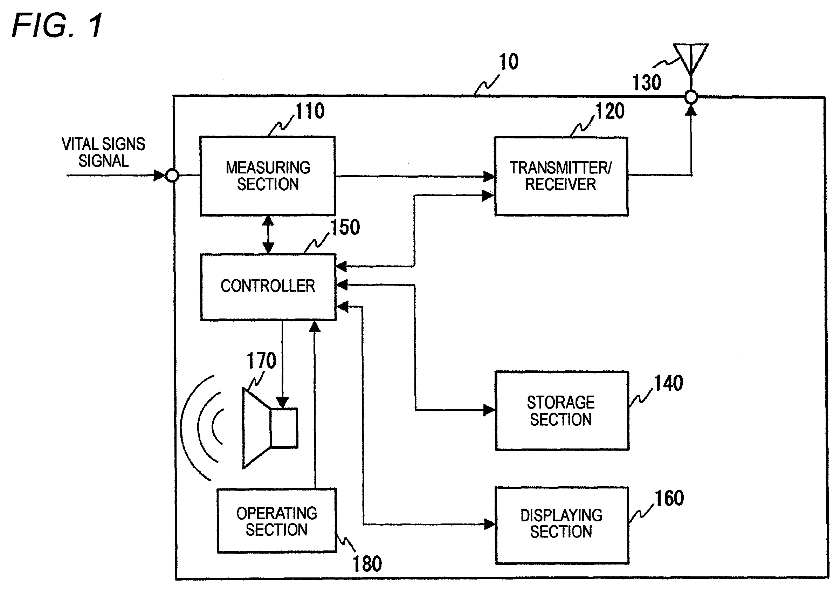

A portable medical apparatus of the embodiment will be described. The portable medical apparatus may be any kind of apparatus as far as it is to be carried by a patient, measures vital signs parameters (the blood pressure, the heart rate, the respiratory rate, an electrocardiogram (including lead waveforms), the arterial oxygen saturation, the concentration of carbon dioxide, and the like), and displays measurement values and waveforms. In the following description, a medical telemeter 10 is assumed as an example of the portable medical apparatus. Fig. 1 is a block diagram showing the configuration of the medical telemeter 10 of the embodiment.

The medical telemeter 10 acquires vital signs signals from various sensors which are attached to the patient, and obtains waveforms and values of a plurality of vital signs parameters based on the vital signs signals. The medical telemeter 10 transmits and receives data to and from a central monitor which is not shown, by means of wireless communication.

The medical telemeter 10 is an apparatus having a size and weight which allow the patient to carry (grasp) the medical telemeter in mainly a hospital. The medical telemeter 10 includes a measuring section 110, a transmitter/receiver 120, an antenna 130, a storage section 140, a controller 150, a displaying section 160, a sound emitter 170, and an operating section 180.

The measuring section 110 is electrically connected to electrodes and transducers which are attached to the patient, and acquires vital signs signals. The measuring section 110 then performs signal processing (noise filtering, amplification, and the like) on the vital signs signals to calculate measurement values and waveforms of the vital signs parameters. Here, the vital signs parameters are, for example, an electrocardiogram, the respiratory rate, the respiratory waveform, the blood pressure, the pulse rate, the pulse wave, the arterial oxygen saturation, the concentration of carbon dioxide, and the like. A part of the processing of the measuring section 110 is realized by reading and executing programs by a CPU (Central Processing Unit) which is not shown. The measuring section 110 supplies the calculated measurement values and waveforms of the vital signs parameters to the controller 150 and the transmitter/receiver 120.

The transmitter/receiver 120 is a processing section which transmits and receives data to and from the central monitor which is not shown, through the antenna 130. For example, the transmitter/receiver 120 performs data transmission and reception with respect to the central monitor by means of a wireless communication process which is conducted through access points in an in-hospital network. The transmitter/receiver 120 transmits the measurement values and waveforms of the vital signs parameters which are measured by the measuring section 110, to the central monitor.

The antenna 130 is an antenna device which is used in the communication process with respect to an access point (not shown).

The storage section 140 is a storage device which stores various data. The various data include data such as histories of measurement values and waveforms of the vital signs parameters which are measured by the measuring section 110, and programs which are used in the control of the medical telemeter 10. The storage section 140 may be a memory device or a hard disk drive, or a USB (Universal Serial Bus) memory which is configured so as to be attachable to and detachable from the medical telemeter 10. The storage section 140 has a concept including also a temporary storage device (e.g., a cache memory) which is used by the CPU.

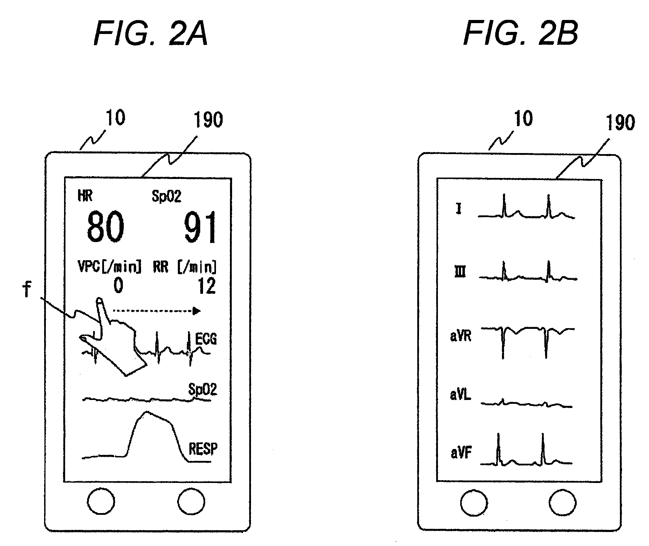

The controller 150 controls the medical telemeter 10, reads various programs from the storage section 140, and executes the programs. The function of the controller 150 is realized by execution of the programs by the CPU disposed in the medical telemeter 10, and processes performed by circuits peripheral to the CPU. The controller 150 switches the display on the displaying section 160 in accordance with an operation which is performed by the user on the operating section 180. The display control will be described later in detail with reference to Figs. 2A and 2B, etc.

The displaying section 160 is a displaying device which is disposed on a housing of the medical telemeter 10, such as an LCD (Liquid Crystal Display) or a CRT (Cathode Ray Tube). Measurement values (the respiratory rate and the like), biological waveforms, and the like of various vital signs parameters are displayed on the displaying section 160. Here, a first screen on which information of at least a part of the vital signs parameters that are measured by the measuring section 110 is displayed, and a second screen on which information at least a part of which is different from that of the first screen with respect to a plurality of vital signs parameters is displayed. Examples of the first and second screens will be described with reference to Figs. 2A and 2B, etc.

The sound emitter 170 is a speaker which emits an electronic sound, a message, and the like to the patient. For example, the sound emitter 170 outputs an alarm notifying an abnormality of the condition of the patient, under the control of the controller 150.

The operating section 180 is an input interface which receives an input (user operation) performed by the user (mainly the patient, the nurse, or the like). The operating section 180 is configured by, for example, buttons disposed on the housing of the medical telemeter 10. The user inputs operation instructions for starting and ending the measurement of various measurement parameters (for example, the body temperature, and the Sp02), instructions for starting transmission of information of the measured vital signs parameters, instructions for stopping the transmission, and the like, through the operating section 180.

A configuration where the displaying section 160 and the operating section 180 are integrated with each other, i.e., a configuration similar to a touch panel may be employed. In the following, the description is made assuming that the displaying section 160 and the operating section 180 are configured as a touch panel.

The display control on the displaying section 160 (touch panel) will be described with reference to Figs. 2A and 2B. Figs. 2A and 2B are conceptual views showing the display control of the medical telemeter 10 of the embodiment.

The touch panel 190 is configured by integrating the displaying section 160 and operating section 180 which have been described above, with each other. In the figures, in order to facilitate the understanding, the finger f of the user is drawn in a size which is smaller than the actual size. Fig. 2A shows the first screen (default screen) which is displayed on the touch panel 190. In the example shown in Fig. 2A, the heart rate, the arterial oxygen saturation (SpO2), the VPC, the respiratory rate, and three waveforms (an electrocardiogram, the SpO2, and the respiration) are displayed as vital signs parameter information.

When the user wishes to refer to information which is different from that of the first screen, the user performs a predetermined operation (user operation) by using the finger f. The predetermined operation (user operation) includes, for example, at least one of a tap, a double tap, a flick, a swipe, a shake, a pinch in, and a pinch out.

The controller 150 detects the operation which is performed on the touch panel 190 during the display of the first screen. In response to the operation, then, the controller 150 switches the screen to be displayed on the touch panel 190, to the second screen. The second screen displays vital signs parameter information which is different from that of the first screen. The different vital signs parameter information may be information of vital signs parameters which are different in kind from those displayed on the first screen, or long-term waveforms and the like of the same vital signs parameters.

Fig. 2B shows an example of the second screen which is displayed after an operation performed by using the finger f. On the second screen, displayed is information that is different from that displayed on the first screen, i.e., lead waveforms (lead I, lead II, lead III, and unipolar limb leads (lead aVR, lead aVL, and lead aVF)).

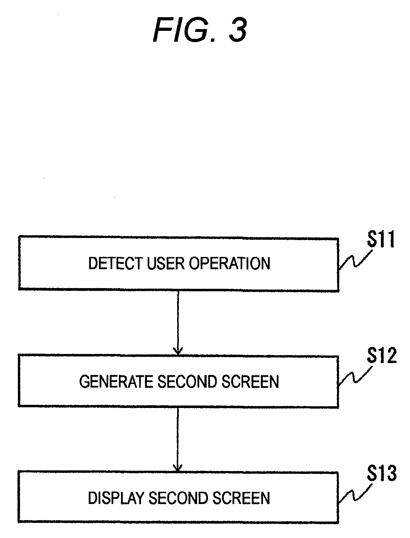

Referring to Fig. 3, the operation of the controller 150 will be again described. Fig. 3 is a flowchart showing the operation of the controller 150 during a period when the first screen is displayed. The controller 150 detects a user operation (for example, a tap or a flick) performed on the touch panel 190 (S11). In the case where the user operation is detected, the controller 150 reads measurement values and the like of vital signs parameters which are to be displayed on the second screen, from the storage section 140, and generates the second screen on which information (waveforms and measurement values) different from that of the first screen is displayed (S12). Then, the controller 150 causes the second screen to be displayed on the touch panel 190 (S13). The operation of the controller 150 during a period when the second screen is displayed is approximately identical with the above. In the case where a user operation is detected, namely, the controller 150 may generate a display screen which is to be next displayed, and switch the display.

In the above, the medical telemeter 10 (portable medical apparatus) of the embodiment has been described. Then, effects of the medical telemeter 10 (portable medical apparatus) will be described. In the above-described medical telemeter 10, the first and second screens which display different information with respect in response to a user operation. Therefore, necessary vital signs parameter information can be accessed simply by a simple operation (for example, a tap or a flick) without performing complicated setting.

In the case where, in a similar manner as in a usual medical telemeter, a process of changing the display screen is performed by changing the setting, when an operation of recovering the setting is forgotten to be performed, there may arise a case where desired vital signs parameters cannot be checked. In the medical telemeter 10 of the embodiment, by contrast, the screen can switched simply by a simple user operation (for example, a tap or a flick), and therefore it is possible to resolve such difficulty of use.

Although, in the above, the example in which the display is switched by an operation performed on the touch panel 190 has been described, it is a matter of course that a configuration where the display is switched by performing a single push on the operating section 180 configured by buttons or the like may be employed.

Various application examples of the display control of the above-described medical telemeter 10 may be contemplated. Hereinafter, application examples will be described.

(Application Example 1)

Application Example 1 is characterized in that priorities are associated with a plurality of vital signs parameters, respectively, and the configurations of the first and second screens are determined in accordance with the priorities. Hereinafter, the example will be described in detail.

Examples of waveforms and measurement values which can be acquired (measured) by the measuring section 110 are as follows :

[waveforms] = electrocardiogram waveforms (eight waveforms), a waveform of the arterial oxygen saturation, and the respiratory waveform; and

[measurement values] = the heart rate (HR), the VPC (Ventricular, Premature Contraction), ST(I), ST(II), ST(III), ST(aVR), ST(aVL), ST(aVF), ST(Va), ST(Vb), the SpO2, the pulse rate (PR), the respiratory rate (RR), and the concentration of carbon dioxide ($CO_2$).

As described above, a wide variety of waveforms and measurement values can be acquired by the measuring section 110, and are hardly displayed at a time on the screen of the medical telemeter 10. Therefore, the doctor, the nurse, or the like previously sets waveforms and measurement values which are to be preferentially displayed. For example, it is assumed that the heart rate, the arterial oxygen saturation (SpO2), the VPC, and three waveforms (ST(II), the SpO2, and the respiration) are allocated with "Priority = High," and the other waveforms and measurement values are allocated with "Priority = Low." For example, the priorities may be set through a setting screen which is not shown.

The controller 150 generates the first screen that displays information of the vital signs parameters to which "Priority = High" is allocated. In the application example, namely, the first screen is a screen which is preferentially displayed. Fig. 2A shows the first screen which is generated in accordance with the above-described allocation of the priorities. When information of vital signs parameters which are to be preferentially displayed is allocated with a higher priority as described above, necessary information can be preferentially accessed.

In the case where a user operation (for example, a tap or a flick) is performed in the state of Fig. 2A, information of the vital signs parameters (the lead waveforms (ST(I) to ST(III), ST(aVR), ST(aVL), ST(aVF))) to which "Priority = Low" is allocated is displayed as shown in Fig. 2B.

Although, in the above, the switching between the first screen and the second screen has been described, also switching among three or more screens may be similarly performed with considering priorities. Alternatively, also a configuration where priorities are set in the unit of a screen, and vital signs parameters are set for each screen may be employed. For example, ST(II), the SpO2, and the respiratory rate (RR) may be displayed on a first screen having a high priority, lead waveforms (ST (I), ST(II), ST(III), ST(aVR), ST(aVL), and ST(aVF)) may be displayed on a second screen having a medium priority, and parameters related to respiration may be displayed on a third screen having a low priority. In this case, a part of vital signs parameters may be displayed on the screens (for example, ST(II) may be displayed on all the screens).

The priorities may be set for each hospital or medical department. Alternatively, the priorities may be set in accordance with the previous diseases of the patient. In the case where the medical telemeter 10 is used for a patient with cardiac disease, for example, it is contemplated that an electrocardiogram waveform and the heart rate are allocated with a high priority. Default priorities may be set for each medical department. In the case where the medical telemeter 10 is used in a cardiovascular department, for example, priorities of vital signs parameters such as an electrocardiogram are set high by default. In the case where the medical telemeter 10 is used in a pulmonology department, by contrast, priorities of vital signs parameters such as the respiratory waveform are set high by default.

Alternatively, the controller 150 may control so that, when the medical telemeter 10 is powered ON or when a power saving mode is canceled, the first screen on which information having a high priority is to be displayed is always displayed. In the above-described example, the controller 150 controls so that, when the medical telemeter 10 is powered ON or when a power saving mode is canceled, the information (first screen) shown in Fig. 2A is displayed by default. After the start of use, therefore, the user can immediately access information having a high priority.

When a screen on which information having a high priority is displayed distinctively from that on which information having a low priority, the user can recognize more clearly information.

(Application Example 2)

Application Example 2 is characterized in that the screens are switched in accordance with normality/abnormality of the measurement value or waveform of each vital signs parameter. Hereinafter, the operation of the application example will be described in points different from the above-described operations.

The controller 150 determines normality/abnormality with respect to the state of each vital signs parameter. For example, the controller 150 determines periodically or always whether measurement values such as the pulse rate and the arterial oxygen saturation are within the respective normal ranges or not, whether electrocardiogram waveforms are normal or not, and the like. That is, the controller 150 performs determining processes which are conducted for a vital alarm and a technical alarm in a usual medical telemeter. The controller 150 identifies a vital signs parameter which is or was abnormal (in the following description, for the sake of convenience, "vital signs parameter which is or was abnormal" is referred to as "vital signs parameter which is abnormal," but "vital signs parameter which was abnormal" should not be excluded when interpreting the term).

Similarly with the above-described example, the controller 150 detects a user operation which is performed during the display of the first screen. The controller 150 identifies a vital signs parameter which is in an abnormal state. Here, the abnormal state includes abnormality of the biological mechanism (for example, cardiac disease), and also abnormality such as detachment of an electrode (abnormality due to the setting of the apparatus or sticking of electrodes). The controller 150 generates the second screen containing information of the vital signs parameter which is abnormal, and related vital signs parameter information, and causes the second screen to be displayed on the touch panel 190 (displaying section 160).

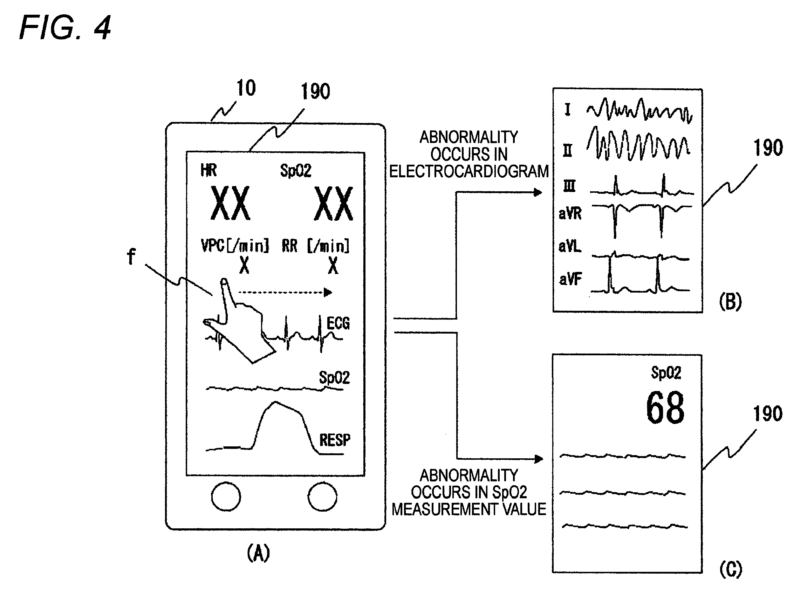

Fig. 4 is a conceptual view showing an example of the display control in Application Example 2. In (A) of Fig. 4, the heart rate, the arterial oxygen saturation (SpO2), the VPC, the respiratory rate, and three waveforms (an electrocardiogram, the SpO2, and the respiration) are displayed on the first screen. It is assumed that, in this state, the controller 150 detects an operation performed by using the finger f. The controller 150 determines periodically or always a vital signs parameter which is abnormal, independently from the detection of a user operation.

Here, it is assumed that the electrocardiogram waveform is determined to be abnormal. The controller 150 specifies the electrocardiogram waveform and information related to the electrocardiogram waveform, and causes them to be displayed on the second screen. The conceptual view in (B) of Fig. 4 shows the second screen in the case where abnormality occurs in the electrocardiogram waveform. In the screen, lead waveforms are displayed as information related to the electrocardiogram waveform. Of course, also the electrocardiogram waveform may be displayed in addition.

On the other hand, it is assumed that the SpO2 measurement value is determined to be abnormal. The controller 150 specifies information related to the SpO2, and causes it to be displayed on the second screen. The conceptual view in (C) of Fig. 4 shows the second screen in the case where abnormality occurs in the SpO2 measurement value. In the screen, a long-term waveform and measurement value of the SpO2 are displayed as information related to the SpO2 measurement value.

In the case where there is no vital signs parameter which is abnormal, the controller 150 may cause information related to a vital signs parameter having a low priority to be displayed as in Application Example 1, or may cause information other than vital signs parameters which are currently displayed, to be displayed.

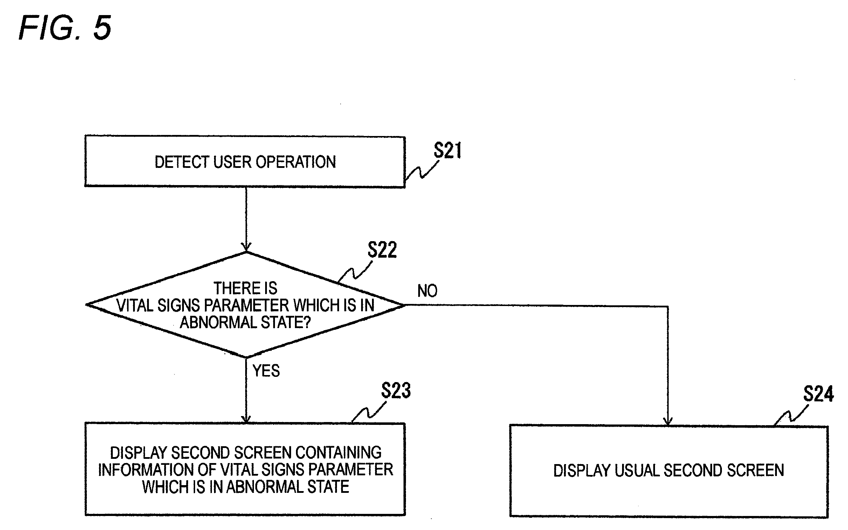

Referring to Fig. 5, the operation of the controller 150 in Application Example 2 will be again described. The controller 150 sequentially determines whether the measurement values and waveforms of vital signs parameters are in an abnormal state or not. The controller 150 detects a user operation during the display of the first screen (S21). The controller 150 determines whether there is a vital signs parameter which is in an abnormal state or not (S22). If there is a vital signs parameter which is in an abnormal state (S22: Yes), the controller 150 generates the second screen containing information of the vital signs parameter which is in an abnormal state, or information related to the vital signs parameter, and causes the second screen to be displayed (S23). By contrast, if there is not a vital signs parameter which is in an abnormal state (S22: No), the controller 150 generates the second screen which displays arbitrary information that is different from that of the first screen, and causes the second screen to be displayed (S24).

In the case where a vital signs parameter has been once abnormal, and thereafter continues to be normal for a predetermined time period or longer, it is preferable to treat the vital signs parameter as normal. In other words, the controller 150 excludes a vital signs parameter in which a predetermined time period or longer has elapsed after the parameter becomes a normal state, from the display targets of the second screen. When the measurement value of the SpO2 has been once abnormal, and thereafter is normal for 10 minutes or longer, for example, the measurement value of the SpO2 is treated as normal, and excluded from the display targets of the second screen. Therefore, a state where necessary information can be adequately checked is attained without being affected by a considerably previous state.

Moreover, the controller 150 may perform normality/abnormality determination on vital signs parameters at all times including a period of the power saving mode, and cause information of a vital signs parameter which is abnormal, to be displayed on the first screen. In other words, the controller 150 dynamically changes the priorities described in the paragraph of Application Example 1, in accordance with abnormality determination in vital signs parameters. Therefore, the users (doctors or the like) can immediately know information of a vital signs parameter which is abnormal (or was abnormal) at a timing such as when the power saving mode is canceled. Namely, an abnormality of the patient can be immediately known without performing even a user operation.

Then, effects of the medical telemeter 10 (portable medical apparatus) of Application Example 2 will be described. As described above, information of a vital signs parameter which is in an abnormal state is displayed on the second screen. Therefore, the doctor or the nurse is enable to refer detailed information related to the abnormal state, simply by performing a simple operation when abnormality occurs in the patient.

(Application Example 3)

Application Example 3 is characterized in that the display is switched depending on the coordinates where a process is performed on the displaying section 160 (touch panel 190). Hereinafter, the operation of the application example will be described in points different from the above-described operations.

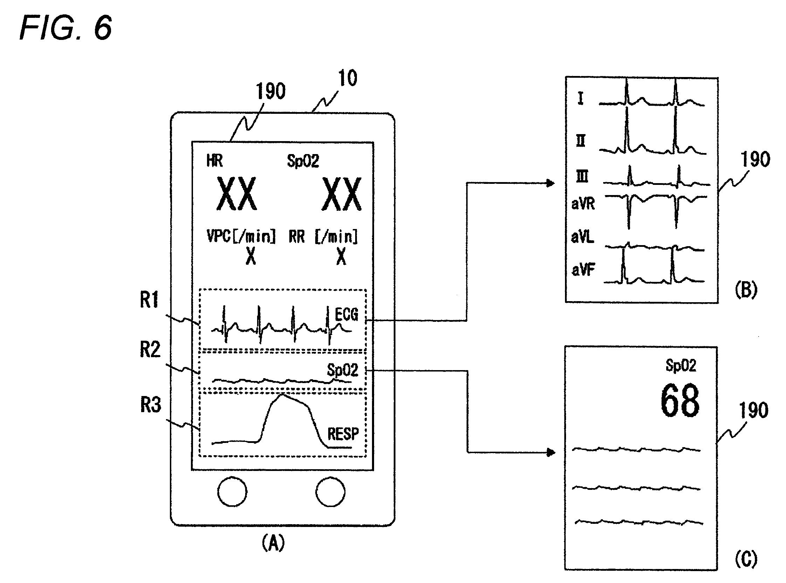

In the conceptual view of (A) of Fig. 6, an example of the first screen is shown. The controller 150 specifies the display contents of the second screen based on the coordinates where a user operation is performed on the touch panel 190. In the example shown in (A) of Fig. 6, regions R1 to R3 are disposed.

The controller 150 identifies the region where a user operation is performed. Then, the controller 150 specifies vital signs parameter information to be displayed on the second screen, depending on the region where the user operation is performed, and the coordinates where information of the vital signs parameters is displayed. In the case where a user operation is performed in the region R1 (region where an electrocardiograms is displayed), for example, the controller 150 generates the second screen in which, as shown in (B) of Fig. 6, information related to the electrocardiogram waveform (waveforms of leads shown in (B) of Fig. 6) is displayed. Alternatively, in the case where a user operation is performed in the region R1 (region where an electrocardiograms is displayed), the controller 150 may generate the second screen (not shown) where a long-term waveform and measurement values of the electrocardiogram waveform are displayed. In the case where a user operation is performed in the region R2 (region where the SpO2 is displayed), similarly, the controller 150 generates the second screen in which, as shown in (C) of Fig. 6, information related to the SpO2 (for example, measurement values and long-term waveform of the SpO2) is displayed. In the case where a user operation is performed in the region R3 (region where the respiratory waveform is displayed), the controller 150 generates the second screen (not shown) where information related to respiration (for example, the respiratory waveform and the respiratory rate) is displayed.

Alternatively, the controller 150 may perform the screen control in consideration of both the region where a user operation is performed, and the kind of the user operation which is performed in the region. In the case where a specific user operation (touch) is performed in the region R1 (region where an electrocardiograms is displayed), for example, the controller 150 performs a control so that long-term waveforms of measurement values are displayed. In the case where another user operation (flick) is performed in the region R1 (region where an electrocardiograms is displayed), the controller 150 performs a control so that lead waveforms ((B) of Fig. 6) are displayed.

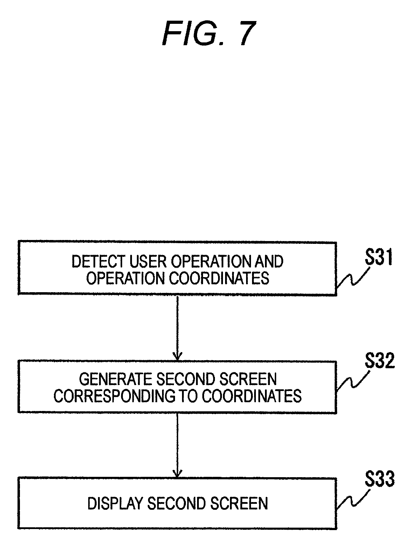

Referring to Fig. 7, the operation of the controller 150 in Application Example 3 will be again described. During the display of the first screen, the controller 150 detects a user operation (S31). In this case, the controller 150 detects also the coordinates where the user operation is performed (S31). Then, the controller 150 reads information of a vital signs parameter corresponding to the detected coordinates, from the storage section 140, and generates the second screen (S32). The controller 150 causes the generated second screen to be displayed on the displaying section 160 (touch panel 190) (S33).

The controller 150 may perform the display control of the second screen also in consideration of the kind of the user operation. In the case where a pinch out (operation of widening the interval between two fingers) is performed in the region R1, for example, the controller 150 generates the second screen in which a long-term waveform of the electrocardiogram is displayed. By contrast, in the case where a double tap (operation of striking two times the screen quickly and lightly) is performed in the region R1, the controller 150 generates the second screen in which lead waveforms (leads I to III, aVR, aVL, aVF, and the like) are displayed.

Alternatively, the display control of the second screen is performed in consideration of only the kind of the user operation and without considering the coordinates. That is, the controller 150 may perform the display control of the second screen in accordance with the kind of a user operation. For example, the association described below may be performed. The following association may be changed through an arbitrary setting screen.

```
[pinch in, pinch out = display of long-term waveform of

electrocardiogram]
```

[double tap = display of lead waveforms]
[flick = display of information related to Sp02]
[swipe = display of information related to respiration]
[shake = display of lead waveforms]

Then, effects of Application Example 3 will be described. In the application example, as described above, information to be next displayed is switched depending on the coordinates of a user operation. Therefore, the user (the doctor or the nurse) can immediately access information which is intuitively related to the coordinates where the user operation is performed. Moreover, the medical telemeter 10 performs the display control in consideration the kind of a user operation (for example, a tap or a pinch out), and therefore can provide information more intuitively.

(Application Example 4)

The application example is characterized in that the second screen which takes over the setting of the first screen is generated. Hereinafter, the operation of the application example will be described in points different from the above-described operations.

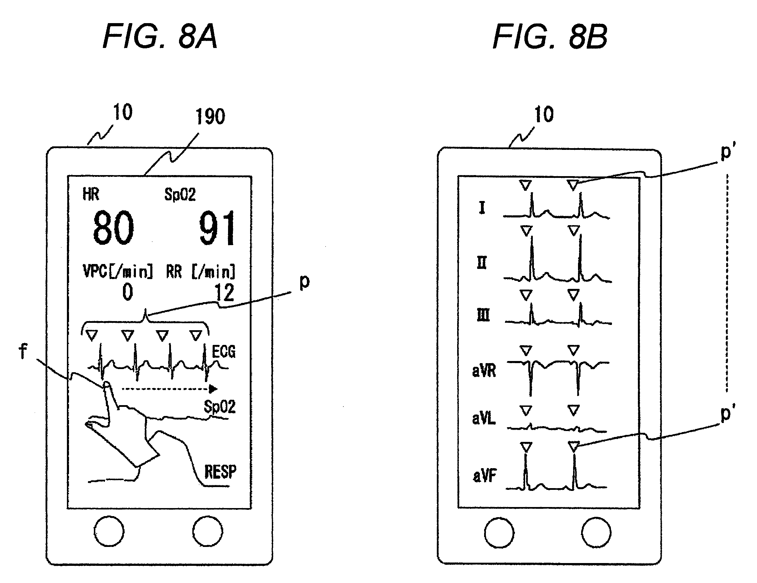

The controller 150 causes the first screen to be displayed at the timing when the power is turned ON. At this time, the controller 150 generates the first screen in accordance with information which is previously set through the setting screen or the like. Fig. 8A is a view showing an example of the first screen which is generated in accordance with the set information.

In the example, the setting is performed so that pacing pulses P are displayed in an electrocardiogram. It is assumed that a user operation is performed in this situation. The controller 150 generates the second screen in a manner similar to Fig. 2B, and causes the second screen to be displayed. At this time, the controller 150 generates the second screen in a state where the setting in which the pacing pulses P are displayed is taken over. Fig. 8B is a view showing an example of the second screen in the case where a user operation is performed on the screen of Fig. 8A. As illustrated, the controller 150 generates the second screen in which pacing pulses P' are displayed together with lead waveforms, and causes the second screen to be displayed.

In the medical telemeter 10 of the application example, even in the case where the screen is switched, as described above, the display setting such as the display of pacing pulses is taken over. Even in the case where the screen is switched, therefore, the user can continuously refer necessary information.

Although the presently disclosed subject matter has been specifically described based on the embodiment, the presently disclosed subject matter is not limited to the above-described embodiment, and it is a matter of course that various changes can be made without departing from the spirit of the presently disclosed subject matter.

Although, in the above, the description has been made with focusing attention on the transition from the first screen to the second screen, it is a matter of course that the above described techniques (Embodiment 1 and Application Examples 1 to 4) can be applied also to a configuration where there are three or more screens for displaying vital signs parameters.

A part or all of the above-described processes in the measuring section 110 and the controller 150 may be realized as computer programs which are executed in the medical telemeter 10 (portable medical apparatus).

The programs may be stored in a non-transitory computer readable medium of any one of various types, and then supplied to the computer. The non-transitory computer readable medium includes tangible storage media of various types. Examples of the non-transitory computer readable medium are a magnetic recording medium (for example, a flexible disk, a magnetic tape, and a hard disk drive), a magneto-optical recording medium (for example, a magneto-optical disk), a CD-ROM (Read Only Memory), a CD-R, a CD-R/W, a semiconductor memory (for example, a mask ROM, a PROM (Programmable ROM), an EPROM (Erasable PROM), a flash ROM, and a RAM (Random Access Memory)). Alternatively, the programs may be supplied to the computer by means of a transitory computer readable medium of any one of various types. Examples of the transitory computer readable medium include an electrical signal, an optical signal, and an electromagnetic wave. The transitory computer readable medium can supply the programs to the computer through a wired communication path such as a metal wire or an optical fiber, or a wireless communication path.

In the above-described portable medical apparatus, the first and second screens which display different kinds of information are displayed for a plurality of vital signs parameters are switched in response to a user operation. According to the configuration, information of a necessary vital signs parameter can be accessed simply by a simple operation (for example, a tap or a flick) without performing complicated setting.

According to an aspect of the presently disclosed subject matter, there may be provided a portable medical apparatus, program, and method of displaying vital signs information in which information of various vital signs parameters can be checked without performing complicated setting.

**Claims**

1. A portable medical apparatus comprising:

   a measuring section which is configured to measure a plurality of vital signs parameters based on vital signs signals of a patient;
   an operating section which is configured to receive a user operation;
   a displaying section which is configured to display:

      a first screen for displaying first information of at least a part of the plurality of vital signs parameters; and
      a second screen for displaying second information of at least a part of the plurality of vital signs parameters, the second information at least a part of which is different from the first information; and
      a controller which is configured to switch the first screen and the second screen in the displaying section, based on the user operation.

2. The portable medical apparatus according to claim 1, wherein
   priorities are set to the plurality of vital signs parameters, respectively, and
   the controller is configured to specify, based on the priorities, the first information to be preferentially displayed on the first screen.

3. The portable medical apparatus according to claim 2, wherein
   the controller is configured to control the displaying section to display the first screen when the portable medical apparatus is powered ON or when a power saving mode of the portable medical apparatus is canceled.

4. The portable medical apparatus according to claim 2 or 3, wherein
   the controller is configured to change the priorities of the plurality of vital signs parameters in accordance with abnormality determination in the plurality of vital signs parameters.

5. The portable medical apparatus according to claim 1, wherein,
   when the operating section receives the user operation while the first screen is displayed in the displaying section, the controller is configured to generate the second screen for displaying information of a vital signs parameter which is or was in an abnormal state, or information of a vital signs parameter related to the abnormal state.

6. The portable medical apparatus according to claim 5, wherein
   the controller is configured to exclude a vital signs parameter in which a predetermined time period or longer has elapsed after transition from an abnormal state to a normal state, from a vital signs parameter information of which is to be displayed on the second screen.

7. The portable medical apparatus according to any one of claims 1 to 6, wherein
   the operating section and the displaying section are integrated with each other to be a touch panel.

8. The portable medical apparatus according to claim 7, wherein,
   while the first screen is displayed in the displaying section, based on coordinates where the user operation is performed on the touch panel, and display coordinates of the first information on the first screen, the controller is configured to specify the second information to be displayed on the second screen.

9. The portable medical apparatus according to claim 1, wherein
   the controller is configured to control a display of the second screen in accordance with a kind of the user operation.

10. The portable medical apparatus according to any one of claims 1 to 9, wherein
    the user operation includes at least one of a tap, double tap, flick, swipe, shake, pinch in, and pinch out performed on one of the first and second screens.

11. A program which causes a computer to execute:

    a measuring step of measuring a plurality of vital signs parameters based on vital signs signals of a patient; and
    a controlling step of, based on a user operation which is input by a user, switching a first screen for displaying first information of at least a part of the plurality of vital signs parameters, and a second screen for displaying second information of at least a part of the plurality of vital signs parameters, the second information at least a part of which is different from the first information.

12. A method of displaying vital signs information, the method comprising:

measuring a plurality of vital signs parameters based on vital signs signals of a patient;
receiving a user operation;
displaying: a first screen for displaying first information of at least a part of the plurality of vital signs parameters; and a second screen for displaying second information of at least a part of the plurality of vital signs parameters, the second information at least a part of which is different from the first information; and
switching the first screen and the second screen, based on the user operation.

13. A program which causes a computer to execute the method according to claim 12.

14. A non-transitory computer-readable recording medium in which the program according to claim 13 is stored.

*FIG. 1*

## FIG. 2A

HR      SpO2

80    91

VPC[/min]  RR [/min]

0     12

f

ECG

SpO2

RESP

## FIG. 2B

I

III

aVR

aVL

aVF

EP 3 086 250 A2

# FIG. 3

| | |
|---|---|
| DETECT USER OPERATION | S11 |

↓

| | |
|---|---|
| GENERATE SECOND SCREEN | S12 |

↓

| | |
|---|---|
| DISPLAY SECOND SCREEN | S13 |

*FIG. 4*

HR   SpO2

**XX**   **XX**

VPC[/min]  RR [/min]
  X            X

f

ECG

SpO2

RESP

(A)

ABNORMALITY
OCCURS IN
ELECTROCARDIOGRAM

I
II
III
aVR
aVL
aVF

(B)

190

SpO2

**68**

(C)

190

ABNORMALITY
OCCURS IN SpO2
MEASUREMENT VALUE

EP 3 086 250 A2

## FIG. 5

DETECT USER OPERATION —— S21

THERE IS
VITAL SIGNS PARAMETER WHICH IS IN
ABNORMAL STATE? —— S22

NO

YES

DISPLAY SECOND SCREEN CONTAINING
INFORMATION OF VITAL SIGNS PARAMETER
WHICH IS IN ABNORMAL STATE —— S23

DISPLAY USUAL SECOND SCREEN —— S24

FIG. 6

(A)

(B)

(C)

EP 3 086 250 A2

# FIG. 7

| DETECT USER OPERATION AND OPERATION COORDINATES | S31 |
|---|---|

| GENERATE SECOND SCREEN CORRESPONDING TO COORDINATES | S32 |
|---|---|

| DISPLAY SECOND SCREEN | S33 |
|---|---|

FIG. 8A

FIG. 8B

18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005529396 T